# EUROPEAN PATENT APPLICATION

(11) **EP 1 949 882 A2**
(43) Date of publication of application: **30.07.2008**
(21) Application number: 08150674.3
(22) Date of filing: 25.01.2008
(51) Int. Cl.: A61F 15/00

(54) **Protective cover for injured members**

(30) Priority: 29.01.2007 ES 200700180 U
(71) Applicant: Balart Tarrida, Santiago, 08784 Piera (ES); Balart Tarrida, Martin, 08784 Piera (ES)
(72) Inventor: Balart Tarrida, Santiago, 08784 Piera (ES); Balart Tarrida, Martin, 08784 Piera (ES)
(74) Representative: Ponti Sales, Adelaida

(57) **Abstract**

The protective cover (1) for injured limbs comprises a waterproof bag (3) and fastening means (6) of the elastic ribbon (5) that do not occupy the whole surface of the end of the elastic ribbon (5), leaving at least three bands (7a, 7b, 7c) free at both sides of the fastening means (6) and in one end, a first band (7a) for contacting with the cover, a second band (7b) for contacting with the injured member, and a third band (7c) for contacting with the ribbon (5), hence providing an effective and durable fastener, because the fastening means (6) are not exposed to external attacks, due to the fact that the elastic ribbon (5) covers and prevents any contact with the external agents inside the bag containing the injured member and also with the fastening means of the cover itself.

## Description

The present invention refers to a protective cover for injured members, which allows a complete isolation of the fastening system.

### BACKGROUND OF THE INVENTION

There are different kinds of covers for injured members that serve to protect said members from external agents such as air, sand, dust, and most generally, water. All of them are waterproof and are provided with a fastening system to guarantee the waterproofing of the assembly over the injured member. The terms "bag" and "cover" will used be indistinctly through all the document.

One kind of known covers comprises as fastening system a perimeter elastic ribbon independent from the cover, that rolls over the mouth of the cover once it is placed protecting the injured member. This solution presents the drawback that the elastic ribbon can be lost easily. Furthermore, as the ribbon is placed over the mouth by trial and error by the user, it is very difficult to obtain the waterproofing of the cover.

An improvement introduced in the previous cover consists of placing the elastic ribbon over the mouth of the cover fastening it by means of its ends and remaining approximately the upper half of the ribbon in contact with the injured member, and the lower part of the ribbon in contact with the cover.

This improvement permits an use enhancement in non-demanding conditions, but it does not solve the problem in its entirety, because when the use conditions are more severe, the fastening means are affected because they are not protected conveniently from the external agents. Therefore, said fastening means can be worsen easily or can lose temporally its efficiency, such as e.g. when it contacts water, compromising the waterproofing of the cover and the integrity of the injured member.

### DESCRIPTION OF THE INVENTION

The objective of the present invention is to solve said drawbacks, presenting other advantages that will be described hereinafter.

The cover for injured members of the present invention is characterised in that said fastening means of the elastic ribbon do not occupy the whole surface of the end of the elastic ribbon, leaving at least three bands free at both sides of the fastening means and in one end, a first band for contacting with the cover, a second band for contacting with the injured member, and a third band for contacting with the ribbon.

Therefore, an effective and durable fastener is obtained, because the fastening means are not exposed to external attacks, because the elastic ribbon covers and prevents any contact with the external agents inside the bag/cover containing the injured member and also with the fastening means of the cover itself.

The elastic ribbon is really the active component which isolates the injured member from the outside because the lower part of the ribbon is in contact with the cover and the upper part is in contact with the member, conforming a band that guarantees the waterproofing of the assembly. Furthermore, the fastening means are always isolated by the elastic ribbon when it is fastened, because it is covered completely and prevents any contact with the outside, protecting it from any external agent.

The injuries of members that can be protected with said cover are diverse and can be, among others, open injuries by impact or tear, deep scratches or cuts, sunburns, burns caused by fire or chemical causes, severe dermatitis, mycotic or bacterial infections and bone fractures protected by casts.

Advantageously, the perimeter elastic ribbon is attached to the entrance mouth of the cover. Therefore, the elastic ribbon is always in contact with the cover, and the handling of the cover is easier and prevents the loss of the ribbon.

Preferably, the protective cover of the invention comprises a second elastic ribbon placed inside a housing obtained folding inwardly the perimeter edge of the end of the cover corresponding to the mouth inside the cover and joining said perimeter edge to the internal surface.

This second elastic ribbon, which is of a permanently closed kind, allows the mouth to fit the arm just inserting the arm inside it, so that there is no need for the user to hold the cover with any other help when he adjusts the elastic ribbon provided with fastening means.

Furthermore, this second ribbon has a second essential function that consists to prevent, by friction, the mouth from rotating around the arm when a force is applied to adjust the elastic ribbon provided with fastening means.

More preferably, all the permanent attachments and junctions of the cover are realised by thermal bonding, specifically the attachment of one of the ends of the elastic ribbon to the external perimeter of the mouth and the junction of the perimeter edge to the internal surface of the cover.

This is necessary because the thermal bonding permits an intimate junction without any hole, even little, that could permit the entrance of water inside the bag/cover, as it happens with sewed fastenings.

Advantageously, said first, second and third bands have substantially the same width, which provides an overlapping between the zones corresponding to both ends of the elastic ribbon and, therefore, allows the user not to do any precision effort to fasten the elastic ribbon over itself. As corresponding zones it must be understood the first band with the first band, the second one with the second one, etc.

More advantageously, the width of these bands is about 15 mm, sufficiently narrow for the ribbon to be stretched manually and sufficiently wide to obtain with enough probability the overlap between the band necessary to prevent water form penetrating inside the bag.

Finally, said fastening means are two Velcro® strips placed each at one end of said ribbon, the strip corresponding to the end attached to the entrance mouth having a length in the perimeter direction of approximately twice of the strip of the free end, which allows guaranteeing an overlap enough between both Velcro® strips complementary with the length of the perimeter of the entrance mouth.

### BRIEF DESCRIPTION OF THE DRAWINGS

For a better understanding of what has been explained, some drawings are attached in which, diagrammatically and only as a non-limitative example, a practical embodiment is shown.
Fig. 1 is an elevation view of the cover; and
Fig. 2 is an elevation view of the cover covering an injured member.

### DESCRIPTION OF A PREFERRED EMBODIMENT

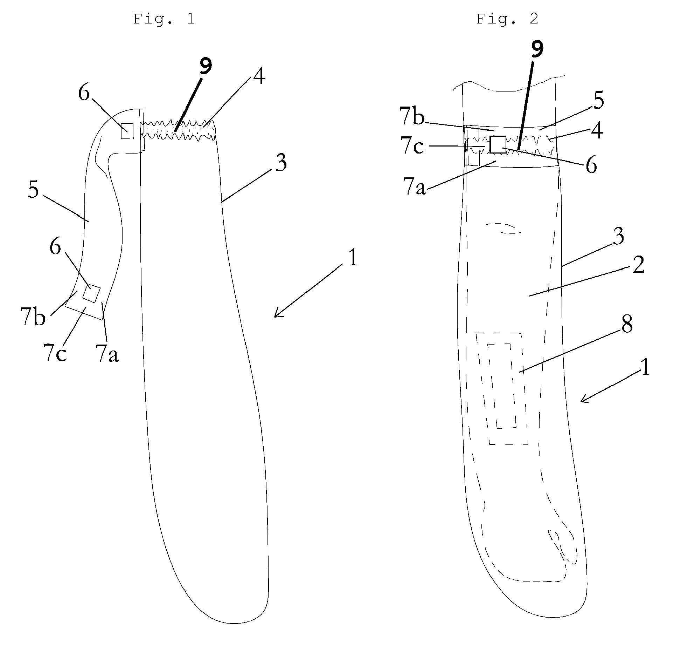

As shown in Fig. 1, the cover 1 to protect a member 2 comprises a cover 3 with an entrance mouth 4 and an elastic ribbon 5 which permits to fasten the cover 1. The elastic ribbon 5 comprises at its ends fastening means 6 to secure the fastening of the ribbon 5. The fastening means 6 occupy a zone of the ribbon 5, leaving free at least three bands 7a, 7b and 7c below it, above it, and by the end, respectively.

In Fig. 2 a member 2 with an injure 8 can be seen, being both covered by a cover 1. Said cover 1 is in its use position and the ribbon 5 surrounds externally the member 2 and the mouth 4, and remains fixed in position thanks to the fastening means 6 that tighten and adjust it suitably. Three free bands can be seen, a first one 7a below the fastening means 6, a second one 7b above it, and a third one 7c at the side of said fastening means 6. The first band 7a is in contact with the cover 1, the second band 7b is in contact with the member 2 and the third band 7c is in contact with the ribbon.

This configuration assures the waterproofing of the cover 1 and the fastening means 6 in any use condition.

As it can be seen also in Fig. 1, the entrance mouth comprises, in this preferred embodiment, a closed elastic ribbon 9 placed inside a housing obtained folding the edge of the bag at the end of the mouth. In Fig. 1, this ribbon is not visible, but its effect can be seen at the entrance mouth, which is wrinkled 9 along the whole perimeter. This housing remains closed by thermal bonding of said edge in the internal surface of the bag or cover. This ribbon is aimed, as in a lot of covers of the state of the art, to allow the cover to be held in the arm or member to which the pressure of this second ribbon is applied. The friction force provided is enough to hold the cover, but not to prevent the entrance of water, as the applicant has proved using several covers available in the market. This is because if only this second ribbon is used to guarantee the waterproofing, the pressure exerted would depend on the diameter of the member or arm. However, the combination of both ribbons, one with open ends and provided with fastening means adjustable in diameter with other permanent ribbon which provides the force enough to hold it when the first one is fastened, guarantees an easy, comfortable and optimal placement for the user.

Another essential feature of this embodiment, that can be seen in Figs. 1 and 2, is that said three bands have substantially the same width, to guarantee the overlap by pairs of the bands corresponding to two ends of the adjustable elastic ribbon. In Fig. 2 a situation in which the elastic ribbon provided with fastening means is perfectly adjusted is shown, i.e. the bands of both ends coincide. This situation is unlikely, specially if the cover is applied to an arm, because the user adjusts it only with one hand. For this reason, it is necessary to provide the ends with a width to permit an overlap enough even in the case of movement between both ends.

Even though a specific embodiment of the present invention is described and shown, it is apparent for a person skilled in the art that variants and modifications can be carried out, or to substitute the details by other technically equivalent ones, without departing from the protection scope defined by the attached claims.

## Claims

1. Protective cover (1) for injured members, comprising a waterproof bag (3) with an entrance mouth (4), an elastic ribbon (5) at the perimeter of said mouth (4), said ribbon (5) comprising fastening means (6) at its ends to adjust the elastic ribbon (5) to the injured member in the zone of the entrance mouth (4), **characterised in that** the fastening means (6) does not occupy the whole surface of the end of the elastic ribbon, leaving at least three bands (7a, 7b, 7c) free at both sides of the fastening means (6) and at its end, a first band (7a) for contacting with the cover, a second band (7b) for contacting with the injured member and a third band (7c) for contacting with the ribbon.

2. Protective cover according to claim 1, **characterised in that** the perimeter elastic ribbon (5) is attached to the entrance mouth (4) of the bag (3).

3. Protective cover according to claim 1, **characterised in that** it comprises a second elastic ribbon (9) placed inside a housing obtained folding inwardly the perimeter edge of the end of the cover corresponding to the mouth (4) inside the cover (3) and joining said perimeter edge to the internal surface of said cover.

4. Protective cover according to claim 2, **characterised in that** said attachment is carried out by thermal bonding.

5. Protective cover according to claim 3, **characterised in that** said junction is carried out by thermal bonding.

6. Protective cover according to claim 1, **characterised in that** said first, second and third bands (7a, 7b, 7c) have substantially the same width.

7. Protective cover according to any of the previous claims, **characterised in that** said same width is about 15 mm.

8. Protective cover according to claim 1, **characterised in that** said fastening means (6) are two Velcro® strips placed each at an end of said ribbon, the strip corresponding to the end attached to the entrance mouth having a length of approximately twice than the strip of the free end.
